# EUROPEAN PATENT APPLICATION

(11) **EP 1 371 366 A1**
(43) Date of publication of application: **17.12.2003**
(21) Application number: 03011017.5
(22) Date of filing: 16.05.2003
(51) Int. Cl.: A61K 31/00, A61K 31/5513, A61K 31/05, A61P 25/00

(54) **Methods for the prevention and/or the treatment of neurological disorders**

(30) Priority: 17.05.2002 EP 02291218; 27.06.2002 EP 02291600; 03.07.2002 US 393108 P
(71) Applicant: Faust Pharmaceuticals, 91198 Gif sur Yvette Cedex (FR)
(72) Inventor: Neuville, Pascal, 67610 La Wantzenau (FR)
(74) Representative: VOSSIUS & PARTNER

(57) **Abstract**

The present invention relates to the use of substrates of an UDP-glucuronosyltransferase (UGT) and salts thereof, for the prevention and /or the treatment of neurological disorders. In preferred embodiment, the present invention relates to the use of substrates of at least an UDP-glucuronosyltransferase (UGT) expressed in brain and salts thereof, for the prevention and /or the treatment of neurological disorders. It further relates to the use of said substrates, and salts thereof, for preventing and/or treating glutamate cytotoxicity, and more specifically of glutamate induced neurological disorders. Additionally, it concerns the use of said substrates, and salts thereof, for making drugs exerting an inhibitory effect on the release of glutamate.

## Description

The present invention relates to the use of substrates of an UDP-glucuronosyltransferase (UGT) and salts thereof, for the prevention and /or the treatment of neurological disorders. In preferred embodiment, the present invention relates to the use of substrates of at least an UDP-glucuronosyltransferase (UGT) expressed in brain and salts thereof, for the prevention and /or the treatment of neurological disorders. It further relates to the use of said substrates, and salts thereof, for preventing and/or treating glutamate cytotoxicity, and more specifically of glutamate induced neurological disorders. Additionally, it concerns the use of said substrates, and salts thereof, for making drugs exerting an inhibitory effect on the release of glutamate.

A large number of studies have established that cellular communication using excitatory amino acids can be transformed into a mechanism of cell destruction.

Glutamate, for example, is the main excitatory neurotransmitter in the nervous system, especially brain and spinal cord, of mammals wherein it is working at a variety of excitatory synapses.

The ubiquitous distribution of glutamate receptors throughout the nervous system proves that glutamate plays a central role in a wide range of physiological as well as pathological events (Watkins J. C., Collingridge G. L., The NMDA receptor, IRL Oxford, 1989). It is for example strongly suggested that it plays a central role in functions such as learning, pattern recognition, and memory (Bliss T. V. P. Collingridge G. L., Nature 361, 31-39, 1993).

Normally extracellular levels of glutamate are elevated only in a brief and spatially localized fashion associated with normal synaptic transmission; however, under pathologic circumstances levels may remain dramatically increased.

Additionally, it has also been known for decades that glutamate is toxic to neurons *in vitro* and *in vivo* and that the function of glutamate receptors, especially glutamate receptors of the N-methyl-D-aspartate ("NMDA") receptor subtype, is crucial in a number of neuronal damages and injuries (Appel S. H., Trends Neurosci. 16, 3-5, 1993). Many neurological disorders involving epileptic seizures and chronic or acute degenerative processes, such as for example Alzheimer's, Huntington's, Parkinson's diseases, multiple sclerosis (MS), amyotrophic lateral sclerosis (ALS), spinal muscular atrophy (SMA), retinopathy, stroke, depression and traumatic brain injury, involve neuronal cell death caused by over-stimulation of the glutamate receptors. Similarly, it has been shown that neuronal injury caused by ischemia after occlusion of cerebral arteries could, at least partially, be mediated by excessive activation of glutamate receptors as in the ischemic brain, extracellular glutamate is elevated rapidly after the onset of ischemia and declines following reperfusion (Davalos et al., 1997, Stroke, 28, 708-710). Other pathologic circumstances associated with dramatic increase of extracellular glutamate levels are hypoxia or hypoglycaemia. Finally, Stephans and Yamamoto (1994, Synapse, 17, 203-209) have shown that drug-induced neurotoxicity, for example neurotoxic effects of methamphetamine (METH) on striatal dopaminergic neurons, could actually be mediated by over-stimulation of the glutamate receptors.

These excessive activations of glutamate receptors, referred to as "glutamate cytotoxicity", are actually associated with the elevation of extracellular glutamate levels. The mechanisms of the elevation of extracellular glutamate include enhanced efflux of glutamate and/or the reduction of glutamate uptake by cells. Thus, it would be desirable to provide a means of protecting affected cells, especially neurons, from glutamate-induced cytotoxicity, and more specifically to provide means of regulating glutamate release and/or uptake by glutamate producing cells.

To this end, it has already been proposed to target the glutamate receptors, mostly the N-methyl-D-aspartate ("NMDA") receptor, present on the targeted cells by inhibiting them by the use of agonist or antagonist specific molecules. Examples of such molecules are anthranilic acid derivatives (see US 5,789,444), Basilen Blue D-3G (Reactive Blue 2) and Cibacron Blue 3GA and 5-adenylylimidodiphosphate (AMPPNP) (see US 6,326,370), NMDA specific antagonists such as ketamine, gluconate (Sakaguchi et al., 1999, Neuroscience, 92, 677-684), dextromophan, or 3-(2-carboxypiperazin-4-yl)-propyl-1-phosphonic acid (Kristensen et al., 1992, Pain, 51:249-253; Eide et al., 1995, Pain, 61,221-228), or the 2-methyl-6-(phenylethynyl)pyridine (MPEP) which is an antagonist of the metabotropic glutamate receptor subtype 5 (mGluR5) (Ossowska et al., 2001, Neuropharmacology, 41, 413-420).

However, widespread use of these compounds is precluded by their undesirable side effects (e.g. psychotomimetic effects, headache, hallucinations, dysphoria or disturbances of cognitive and motor functions). Thus, the available treatment methods are not satisfactory in terms of safety or efficiency for their wide implementation.

Therefore, there is still a need in the provision of improved methods and means for protecting affected cells, and more preferably neurons, from glutamate-induced cytotoxicity.

Similarly, many neurological disorders are associated with elevated levels of neuromediator (or neurotransmiter) release such as for example acetylcholine (Ach), butyrylcholine (BuCh), gamma-aminobutyrate (GABA), serotonin or the catecholamines, in particular dopamine or ATP.

In mammals, glucuronidation represents a major metabolic pathway which enhances the detoxification and elimination of many lipophilic drugs, pollutants, xenobiotics and endogenous compounds by converting them in less biologically available, and thus less active, glucuronidated substances exhibiting higher polarity and water-solubility, thus facilitating their transport to excretory organs and subsequent excretion in urine or bile (for a review, see Ritter, 2000, Chemico-Biological Interactions, 129, 171-193). The reaction is catalyzed by UDP-glucuronosyltransferases (UGTs). This multigenic family of enzymes transfer the glucuronic acid moiety of UDP-glucuronic acid to structurally unrelated compounds through hydroxyl (alcoholic, phenolic), carboxyl, sulfuryl, carbonyl and amino (primary, secondary or tertiary) linkages (for review, see Tukey and Strassburg, 2000, Annu. Rev. Pharmacol., Toxicol., 40, 581-616). Initially, glucuronidation has been considered to represent a metabolic pathway performed mainly in the liver. However, multiple studies have further indicated that UGT activity was also present in human intestinal, kidney, colon tissue and brain where this enzyme is believed to take part actively in the defence of the organism against potentially reactive hydroxylated molecules which can reach the organ (Suleman et al., 1998, Archives of Biochemistry and Biophysics, 358, 1, 63-67 ; Gradinaru et al., 1999, Neurochemical Research, 24, 995-1000).

The inventors have now shown that the UGT activity *in vivo*, and more specifically in brain, can be turned away and used for defining a new class of compounds for the treatment and/or prevention of acute and chronic neuromediator-related diseases or conditions, particularly neurological diseases. The present invention is a pharmacological alternative to previously described methods implementing compounds, such as competitive and non-competitive glutamate antagonists or agonists, gangliosides and growth factors. In special embodiments, the present invention provides a new class of compounds which can be used as pharmacological tools for the modulation of neuromediator cellular release and cytotoxicity, preferably neurotoxicity, and which allows the possible treatment and/or prevention of many neurological disorders involving epileptic seizures and acute and chronic neurodegenerative diseases, as well as neuronal injury caused by ischemia or neuromediator-related diseases or conditions, wherein said disorders are, at least partially, associated with excessive activation of neuromediator receptors and/or with excessive extracellular neuromediator levels.

First, the invention concerns the use of at least one substrate of an UDP-glucuronosyltransferase (UGT), and salts thereof, for the preparation of a pharmaceutical composition having an inhibitory effect on the extracellular neuromediator release into an individual treated with said composition. According to one special embodiment, said UDP-glucuronosyltransferase (UGT) is expressed in brain.

According to specific embodiment, said UDP-glucuronosyltransferase (UGT) is selected among the group consisting of UDP-glucuronosyltransferase 1A1 (UGT1A1, also termed HUG-Br1 or UGT1.1), UDP-glucuronosyltransferase 1A3(UGT1A3, also termed 1c), UDP-glucuronosyltransferase 1A4 (UGT1A4, also termed HUG-Br2), UDP-glucuronosyltransferase 1A6 (UGT1A6), UDP-glucuronosyltransferase 1A9 (UGT1A9, also termed HLUG P4), UDP-glucuronosyltransferase 2B4 (UGT2B4, also termed Hlug-25, Th-1, h-1, or h-20), UDP-glucuronosyltransferase 2B7 (UGT2B7), UDP-glucuronosyltransferase 2B10 (UGT2B10, also termed h-46), UDP-glucuronosyltransferase 2B15 (UGT2B15, also termed UDPGTh-3,h-3, HLUG-4, or HE 8A), UDP-glucuronosyltransferase 2B17 (UGT2B17) and UDP-glucuronosyltransferase 2B28 (UGT2B28). According to specific embodiment, said UDP-glucuronosyltransferase (UGT) is expressed in brain and is selected among the group consisting of UDP-glucuronosyltransferase 1A6 (UGT1A6) and UDP-glucuronosyltransferase 2B7 (UGT2B7).

According to the present invention, the term "UDP-glucuronosyltransferase (UGT)" refers to enzymes catalyzing the glucuronidation reaction. Said reaction utilizes UDP-glucuronic acid as a cosubstrate for the formation of glucuronides. All of these terms are widely used in the litterature; for a review, please refer to Tukey and Strassburg, 2000, Annu. Rev. Pharmacol. Toxicol., 40, 581-616. The method of choice for analysing the activity of a define UGT is the expression of the corresponding cDNA in a cell line that express minimal levels of the respective protein. More precisely, cells are transfected by standard transient transfection methods or by stable introduction of the cDNA into the genome following selection of the cells with an appropriate antibiotic. Then the cells are homogenized in a buffer containing Tris-HCl and MgCl₂ and subjected to sonication. Cell homogenates are then tested for their ability to glucuronidate series of substrates. The activity and selectivity of the UGT for a substrate could be compared with known specific substrates if available (Remmel and Burchell, 1993, Biochem. Pharmacol., 46, 559-566).

Currently, 15 UGT cDNAs have been identified in human, eight UGT1A proteins encoded by the UGT1A locus and seven proteins encoded by UGT2B genes. "UDP-glucuronosyltransferase (UGT) 1A6" refers to one of the UGT proteins encoded by the UGT1A locus. The human UGT1A6 sequence is presented in SEQ ID NO:1 (Harding et al., 1988, Proc. Natl. Acad. Sci. 85, 8381-8385 ). UGT1A6 is also called PNP-UGT, phenol p I 6.2, UGT1*6, UGT1A06 or HLUGP1. "UDP-glucuronosyltransferase (UGT) 2B7" refers to one of the UGT proteins encoded by the UGT2B locus. The human UGT2B7 sequence is presented in SEQ ID NO:2 (Ritter et al., 1990, J. Biol. Chem., 265, 7900-7906. UGT2B7 is also named Th-2, h-2, Hlug-6, UGT2*7 or Catechol Estrogen UDPGT.

According to the present invention the term "UGT substrate" generally designates both the aglycone substrate (i.e. compound which can be enzymatically converted, or metabolized, by the UGT enzyme as disclosed above) and the glucuronidated substrate (i.e. compound which has been converted by addition of at least one glucuronic acid moiety through active groups).

According to the present invention the term "neuromediator", a synonym of "neurotransmitter", is intended to designate chemicals that transmit information across the junction (synapse) that separates one nerve cell (neuron) from another nerve cell or a muscle. Examples of said neuromediators are acetylcholine (Ach), butyrylcholine (BuCh), gamma-aminobutyrate (GABA), serotonin, norepinephrine, epinephrine, endorphins, or the catecholamines, in particular dopamine or adenosine triphosphate. According to one preferred embodiment, said neuromediator is the glutamate.

According to one embodiment, the UGT substrate of the invention is selected among the group consisting of planar and small phenols, polycyclic aromatic hydrocarbons, and compounds which are structurally related. More specifically, said UGT substrate contains at least one moiety selected in the group consisting in hydroxyl (alcoholic, phenolic, etc...), carboxyl, sulfuryl, carbonyl and amino (primary, secondary or tertiary) moieties.

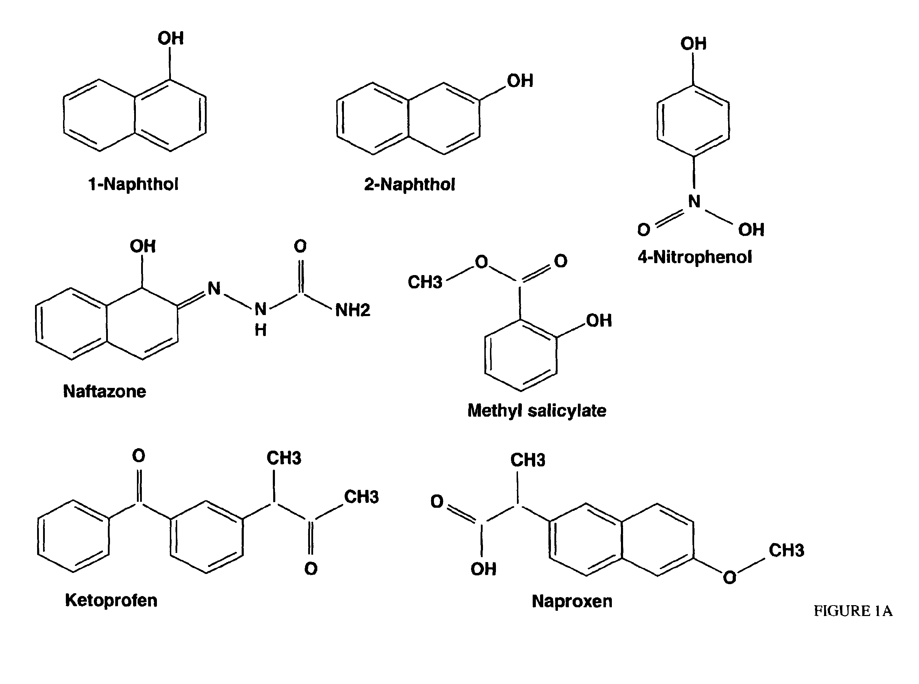

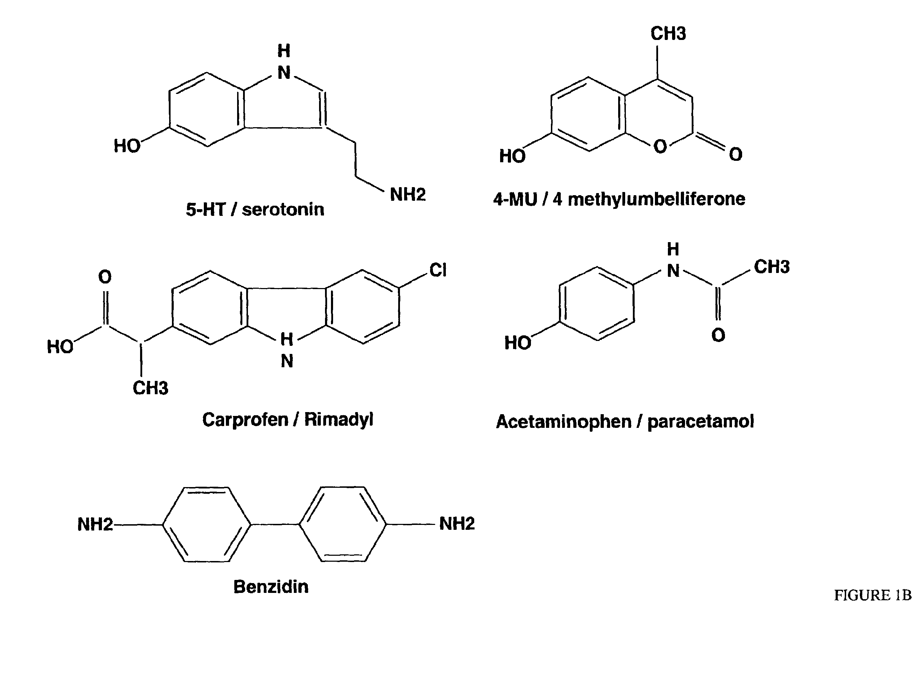

According to a specific embodiment, said substrate is a compound which can be converted by at least the UDP-glucuronosyltransferase 1A6 (UGT1A6) and is selected in the group consisting of 1-Naphthol, 2-Naphthol , 4-nitrophenol , methylsalicylate, ketoprofen , naproxen, 5-OH tryptamine/serotonin, carprofen/rimadyl , acetaminophen/paracetamol ,benzidine, 4-methylumbelliferone (4-MU), silymarin (Venketaramanan et al., 2000, Drug Metab Dispos., 28,1270-3 ; silymarin is a mixture of toxifolin, silichristin, silidianin, silybin A et B, isosilybin A et B) [see Figure 1A and 1B].

According to another embodiment, said substrate is a compound which can be converted by at least the UDP-glucuronosyltransferase 2B7 (UGT2B7) and is selected in the group consisting of transretinoic acid, ASA , AZT , benoxaprofen , benzidine, (benzo(a)pyrene mbs), buprenorphine, carprofen, chloramphenicol, clofibric acid flavenoids quercetin, kaempfenol, cyclosporin, DMXAA, diclofenac, dihydrocodeine DHC, dihydromorphone , mefenamic acid, fenemate NSAID, mycophenolic acid MCPA mofetil, fenoprofen, hydromorphone, ibuprofen, ketoprofen, linoleic acid , lorazepam, losartan, menthol, morphine 3 and morphine 6, nalbufene, nalmefene, naltrindole, nalorphine, naloxone, naltrexone, S-naproxen, norcodeine, normorphone, oxycodone, oxymorphone, pirprofen, propanolol, S-oxazepam, tacrolimus, temazepam, tolcapone, tiaprofenic, valproate, zomepirac, 5-OH tryptamine.

According to a specific embodiment, said substrate is selected in the group consisting of ketoprofen , naproxen, 5-OH tryptamine/serotonin, carprofen/rimadyl and benzidine.

In special embodiments, the UGT substrates of the invention are further substituted with, one to four, identical or different, heteroatoms and/or hetero groups. Examples of said heteroatoms and/or hetero groups are O, H, alkyl or aryl groups CₙHₙ₊₁, with n = 1 to 5, OCH₃, N, halogens (fluorine, chlorine, bromine, or iodine atom), S or any labeling element allowing to visualize said substrates. These substituting atoms or groups, and their uses, are widely known in the art.

The addition salts of the substrates of the invention comprise conventional salt formed from inorganic or organic acids or bases, such as hydrochloric, hydrobromic, sulfuric, phosphoric, nitric, perchloric, fumaric, acetic, sodium, lithium, potassium, magnesium, aluminium, calcium, zinc, ethylenediamine ; formic, benzoic, maleic, tartaric, citric, oxalic, aspartic acid, and alkane-sulfonic acids is even mentioned.

The UGT substrates used according to the invention are commercially available in many commercial catalogues for example in Merck, "Produits chimiques et réactifs" 2002 and Acros Organics "Fine Chemicals" 2002-2003 :

| | |
|---|---|
| 1-Naphtol : | 822289 (Merck) |
| 2-Naphtol : | 822290 (Merck) |
| 4-Nitrophenol : | 820896 (Merck) |
| 5OH Tryptamine/serotonin | 21502-1000 (Acros) |
| 4-methylumbelliferone | 40549-0010 (Acros) |
| All trans retinoic acid | 20734-1000 (Acros) |
| Chloramphenicol | 22792-0250 (Acros) |
| Clofibric acid | 18909-0500 (Acros) |

Further suppliers are :
- Naproxen :: Apranax (FR, Roche) or Naprosyn (US, Roche)
- Carprofen :: Rimadyl (Pfizer)
- Ketoprofen :: Toprec (FR, Aventis) or Orudis (US, Wyeth).

The newly identified inhibitory properties of these UGT substrates make them particularly suitable for treating and/or preventing diseases, conditions and attacks related to deleterious effects of neurotransmitter released, and preferably neurological ones. In one special embodiment, the UGT substrates of the invention are suitable for treating and/or preventing diseases, conditions and attacks related to deleterious effects of glutamate released, and preferably neurological ones.

According to one special embodiment, said diseases, conditions and attacks are related to deleterious effects of neurotransmitter released in excess, and more particularly to deleterious effects of glutamate released in excess.

Thus the present invention further relates to a method for treating and/or preventing neuromediator-evoked cytotoxicity in a patient in need thereof comprising administering to said patient a composition containing a therapeutically effective amount of at least one substrate of an UDP-glucuronosyltransferase (UGT) as defined above and a pharmaceutically acceptable carrier. In special embodiment, said UGT substrate is a compound which can be converted by at least one UDP-glucuronosyltransferase (UGT), and preferably by at least one UDP-glucuronosyltransferase (UGT) expressed in brain.

Preferably, the present invention relates to a method for treating and/or preventing glutamate-evoked cytotoxicity in a patient in need thereof comprising administering to said patient a composition containing a therapeutically effective amount of at least one substrate of an UDP-glucuronosyltransferase (UGT) as defined above and a pharmaceutically acceptable carrier. In special embodiment, said UGT substrate is a compound which can be converted by at least one UDP-glucuronosyltransferase (UGT), and preferably by at least one UDP-glucuronosyltransferase (UGT) expressed in brain.

According to another embodiment, said UGT substrate is selected among the group consisting of planar and small phenols, polycyclic aromatic hydrocarbons, and compounds which are structurally related. More specifically, said UGT substrate contains at least one moiety selected in the group consisting in hydroxyl (alcoholic, phenolic, etc...), carboxyl, sulfuryl, carbonyl and amino (primary, secondary or tertiary) moieties.

According to another embodiment, said substrate is a compound which can be converted by the UDP-glucuronosyltransferase 1A6 (UGT1A6) and is selected in the group consisting of 1-Naphthol, 2-Naphthol , 4-nitrophenol , methylsalicylate, ketoprofen , naproxen, 5-OH tryptamine/serotonin, carprofen/rimadyl , acetaminophen/paracetamol , benzidine, 4-methylumbelliferone (4-MU), silymarin (Venketaramanan et al., 2000, Drug Metab Dispos., 28,1270-3 ; Silymarin is a mixture of toxifolin, silichristin, silidianin, silybin A et B, isosilybin A et B) [see Figure 1].

According to another embodiment, said substrate is a compound which can be converted by the UDP-glucuronosyltransferase 2B7 (UGT2B7) and is selected in the group consisting of transretinoic acid, ASA , AZT , benoxaprofen, benzidine, (benzo(a)pyrene mbs), buprenorphine, carprofen, chloramphenicol, clofibric acid , flavenoids quercetin, kaempfenol, cyclosporin, DMXAA, diclofenac, dihydrocodeine DHC, dihydromorphone, mefenamic acid, fenemate NSAID, mycophenolic acid MCPA mofetil, fenoprofen, hydromorphone, ibuprofen, ketoprofen, linoleic acid , lorazepam, losartan, menthol, morphine 3 and morphine 6, nalbufene, nalmefene, naltrindole, nalorphine, naloxone, naltrexone, S-naproxen, norcodeine, normorphone, oxycodone, oxymorphone, pirprofen, propanolol, S-oxazepam, tacrolimus, temazepam, tolcapone, tiaprofenic, valproate, zomepirac, 5-OH tryptamine.

According to a special embodiment, said substrate is selected in the group consisting of ketoprofen, naproxen, 5-OH tryptamine/serotonin, carprofen/rimadyl and benzidine.

The term "neuromediator-evoked cytotoxicity" or neurotransmitter-evoked cytotoxicity" within the present invention is intended to designate cell toxicity associated with excessive activations of the concerned neuromediator receptors. These terms are well known by the one skilled in the art. More specifically, the "glutamate-evoked cytotoxicity" concerns all affected cells expressing glutamate receptors. According to preferred embodiments, these cells are susceptible to be affected by neuromediators are nervous cells (i.e. neuro-cells), preferably neurons. These affected nervous cells are, for example, present in brain, spinal cord, retina, at the neuro-muscular junction, etc ... "Cytotoxicity" means that the cell functions and/or properties are affected, leading to cell malfunctioning, and finally to cell death.

In a particularly preferred embodiment, the method of the invention is intended for treating and/or preventing neuromediator-evoked neurotoxicity, and even more preferably for treating and/or preventing neurodegeneration (i.e. degeneration of nervous cells).

In another particularly preferred embodiment, the method of the invention is intended for treating and/or preventing glutamate-evoked neurotoxicity, and even more preferably for treating and/or preventing neurodegeneration (i.e. degeneration of nervous cells).

The present invention further relates to a method for modulating the release of at least one neuromediator in a patient comprising administering to said patient a composition containing a therapeutically effective amount of at least one substrate of an UDP-glucuronosyltransferase (UGT) and a pharmaceutically acceptable carrier, wherein said substrate is as detailed above. In special embodiment, said UGT substrate is a compound which can be converted by at least one UDP-glucuronosyltransferase (UGT), and preferably by at least one UDP-glucuronosyltransferase (UGT) expressed in brain.

The present invention further relates to a method for modulating the release of glutamate in a patient comprising administering to said patient a composition containing a therapeutically effective amount of at least one substrate of an UDP-glucuronosyltransferase (UGT) and a pharmaceutically acceptable carrier, wherein said substrate is as detailed above. In special embodiment, said UGT substrate is a compound which can be converted by at least one UDP-glucuronosyltransferase (UGT), and preferably by at least one UDP-glucuronosyltransferase (UGT) expressed in brain.

"Modulating the release of neuromediator" means that the levels of released neuromediator in non treated patient is different from the one observed after his treatment with the substrates of the invention. According to one embodiment, treatment of the patient with the substrates of the invention leads to a negative modulation, preferably to the inhibition, of the neuromediator release by the producing cells, and thus to a decreased neuromediator level in the treated patient compared to the same neuromediator level observed before said treatment.

The present invention further relates to a method for treating and/or preventing disease and/or condition associated with the excessive release of at least one neuromediator in a patient comprising administration to said patient of a composition containing a therapeutically effective amount of at least one substrate of an UDP-glucuronosyltransferase (UGT) and a pharmaceutically acceptable carrier, wherein said substrate is as detailed above. In special embodiment, said UGT substrate is a compound which can be converted by at least one UDP-glucuronosyltransferase (UGT), and preferably by at least one UDP-glucuronosyltransferase (UGT) expressed in brain.

The present invention further relates to a method for treating and/or preventing disease and/or condition associated with the excessive release of glutamate in a patient comprising administration to said patient of a composition containing a therapeutically effective amount of at least one substrate of an UDP-glucuronosyltransferase (UGT) and a pharmaceutically acceptable carrier, wherein said substrate is as detailed above. In special embodiment, said UGT substrate is a compound which can be converted by at least one UDP-glucuronosyltransferase (UGT), and preferably by at least one UDP-glucuronosyltransferase (UGT) expressed in brain.

According to the invention, the terms "treating and/or preventing" refer to a process that is intended to produce a beneficial change in the condition of a mammal, e.g., a human, often referred to as a patient. A beneficial change can, for example, include one or more of: restoration of function, reduction of symptoms, limitation or retardation of progression of a disease, disorder, or condition or prevention, limitation or retardation of deterioration of a patient's condition, disease or disorder. Such therapy can involve, for example, nutritional modifications, administration of radiation, administration of a drug, behavioural modifications, and combinations of these, among others.

According to the invention, "disease and/or condition associated with the excessive release of at least one neuromediator" is intended to designate large number of acute and chronic neuromediator-related diseases or conditions, particularly neurological diseases. It designates more specifically epileptic seizures and acute and chronic neurodegenerative diseases, as well as neuronal injury caused by ischemia or neuromediator-related diseases or conditions, wherein said disorders are, at least partially, associated with excessive activation of neuromediator receptors and/or with excessive extracellular neuromediator levels. Examples are involving chronic or acute degenerative disorders, such as for example Alzheimer's, Huntington's, Parkinson's diseases, multiple sclerosis (MS), amyotrophic lateral sclerosis (ALS), spinal muscular atrophy (SMA), retinopathy, stroke, clinical depression and traumatic brain injury, involve neuronal cell death caused by over-stimulation of the neuromediator receptors, and more specially of the glutamate receptors. Similarly, neuronal injury caused by ischemia or drug-induced neurotoxicity, for example neurotoxic effects of methamphetamine (METH) on striatal dopaminergic neurons, are indications of the methods according to the present invention. Other indications are the neuromediator-related conditions such as for example pain, hormonal balance, blood pressure, thermoregulation, respiration, learning, pattern recognition or memory, or any disorder subsequent to hypoxia or hypoglycaemia, especially when these indications are glutamate-related conditions.

For example, the treatment of epilepsy, clinical depression, amyotrophic lateral sclerosis, spinal muscular atrophy (SMA), Huntington's disease, deleterious effect due to excesses of glutamate released as a result of cerebral accidents of traumatic or other vascular origin will be mentioned.

In the above disclosed embodiments of the invention, most of the compositions and methods were related to the aglycone form of said substrates. Alternatively, the invention further relates to the use of at least one glucuronidated substrate of an UDP-glucuronosyltransferase (UGT) and salts thereof, for the preparation of a pharmaceutical composition having an inhibitory effect on the extracellular neuromediator release into an individual treated with said composition. According to said embodiment, the substrate of an UDP-glucuronosyltransferase (UGT) is understood as being glucuronidated before its administration to the patient in need thereof. Said glucuronidation can be obtained by modifying the aglycone substrate either by chemical or enzymatic way. Chemical glucuronidation can be obtained by using standard chemical method well known by those in the art consisting for example in adding a glucuronide group to the aglycone substrate intended to be used (Lacy and Sainsbury, 1995, Tetrahedron lett., 36,3949-50). Alternatively, the glucuronidated substrate can be obtained by enzymatic glucuronidation using at least one UDP-glucuronosyltransferase (UGT) able to glucuronidate said aglycone substrate. In preferred embodiment said UDP-glucuronosyltransferase (UGT) is naturally expressed in brain, e.g. UDP-glucuronosyltransferase (UGT) 1A6 or UDP-glucuronosyltransferase (UGT) 2B7.

According to one embodiment, said glucuronidated UGT substrate is selected among the group consisting of planar and small glucuronidated phenols, polycyclic aromatic glucuronidated hydrocarbons, and compounds which are structurally related. More specifically, said glucuronidated substrate contains at least one moiety selected in the group consisting in hydroxyl (alcoholic, phenolic, etc...), carboxyl, sulfuryl, carbonyl and amino (primary, secondary or tertiary) moieties linked to glucuronic acid moiety.

According to a specific embodiment, said glucuronidated substrate is selected in the group consisting of 1-Naphthol, 2-Naphthol , 4-nitrophenol , methylsalicylate, ketoprofen , naproxen, 5-OH tryptamine/serotonin, carprofen/rimadyl, acetaminophen/paracetamol,benzidine, 4-methylumbelliferone (4-MU), silymarin (Venketaramanan et al., 2000, Drug Metab Dispos., 28,1270-3 ; Silymarin is a mixture of toxifolin, silichristin, silidianin, silybin A et B, isosilybin A et B) and is further glucuronidated.

According to another embodiment, said glucuronidated substrate is selected in the group consisting of transretinoic acid, ASA , AZT , benoxaprofen, benzidine, (benzo(a)pyrene mbs), buprenorphine, carprofen, chloramphenicol, clofibric acid, flavenoids quercetin, kaempfenol, cyclosporin, DMXAA, diclofenac, dihydrocodeine DHC, dihydromorphone , mefenamic acid, fenemate NSAID, mycophenolic acid MCPA mofetil, fenoprofen, hydromorphone, ibuprofen, ketoprofen, linoleic acid, lorazepam, losartan, menthol, morphine 3 and morphine 6, nalbufene, nalmefene, naltrindole, nalorphine, naloxone, naltrexone, S-naproxen, norcodeine, normorphone, oxycodone, oxymorphone, pirprofen, propanolol, S-oxazepam, tacrolimus, temazepam, tolcapone, tiaprofenic, valproate, zomepirac, 5-OH tryptamine and is further glucuronidated.

The addition salts of the glucuronidated substrates of the invention comprise conventional salt formed from inorganic or organic acids or bases, such as hydrochloric, hydrobromic, sulfuric, phosphoric, nitric, perchloric, fumaric, acetic, sodium, lithium, potassium, magnesium, aluminium, calcium, zinc, ethylenediamine ; formic, benzoic, maleic, tartaric, citric, oxalic, aspartic acid, and alkane-sulfonic acids is even mentioned.

The present invention further relates to a method for treating and/or preventing neuromediator-evoked cytotoxicity in a patient in need thereof comprising administering to said patient a composition containing a therapeutically effective amount of at least one glucuronidated substrate of an UDP-glucuronosyltransferase (UGT) as defined above and a pharmaceutically acceptable carrier.

The present invention further relates to a method for treating and/or preventing glutamate-evoked cytotoxicity in a patient in need thereof comprising administering to said patient a composition containing a therapeutically effective amount of at least one glucuronidated substrate of an UDP-glucuronosyltransferase (UGT) as defined above and a pharmaceutically acceptable carrier.

In a particularly preferred embodiment, said "neuromediator-evoked cytotoxicity" is a neuromediator-evoked neurotoxicity, and even more preferably a neurodegeneration (i.e. degeneration of nervous cells).

In another particularly preferred embodiment, said "glutamate-evoked cytotoxicity" is a glutamate-evoked neurotoxicity, and even more preferably a neurodegeneration (i.e. degeneration of nervous cells).

The present invention further relates to a method for modulating the release of at least one neuromediator in a patient comprising administering to said patient a composition containing a therapeutically effective amount of at least one glucuronidated substrate of an UDP-glucuronosyltransferase (UGT) and a pharmaceutically acceptable carrier, wherein said substrate is as detailed above.

The present invention further relates to a method for modulating the release of glutamate in a patient comprising administering to said patient a composition containing a therapeutically effective amount of at least one glucuronidated substrate of an UDP-glucuronosyltransferase (UGT) and a pharmaceutically acceptable carrier, wherein said substrate is as detailed above.

The present invention further relates to a method for treating and/or preventing disease and/or condition associated with the excessive release of at least one neuromediator in a patient comprising administration to said patient of a composition containing a therapeutically effective amount of at least one glucuronidated substrate of an UDP-glucuronosyltransferase (UGT) and a pharmaceutically acceptable carrier, wherein said substrate is as detailed above.

The present invention further relates to a method for treating and/or preventing disease and/or condition associated with the excessive release of glutamate in a patient comprising administration to said patient of a composition containing a therapeutically effective amount of at least one glucuronidated substrate of an UDP-glucuronosyltransferase (UGT) and a pharmaceutically acceptable carrier, wherein said substrate is as detailed above.

The UGT substrates (including aglycone or glucuronidated form) described in the present invention are administered as a composition containing at least one active compound and a pharmaceutically acceptable carrier. In preparing such a composition, any conventional pharmaceutically acceptable carrier can be utilized. The carrier material can be an organic or inorganic inert carrier material suitable for the selected route of administration. Suitable carriers include water, gelatin, gum arabic, lactose, starch, magnesium stearate, talc, vegetable oils, polyalkylene-glycols, petroleum jelly and the like. Furthermore, the pharmaceutical composition may contain other pharmaceutically active agents. Additional additives such as flavoring agents, preservatives, stabilizers, emulsifying agents, salts for varying the osmotic pressure, buffers and the like may be added in accordance with accepted practices of pharmaceutical compounding. Any conventional form such as tablets, capsules, pills, powders, granules, and the like may be used. Advantageously, they are in the form of tablets, sugar coated tablets, hard gelatin capsules, capsules, granules, for oral administration, or solutions or suspensions for administration via an injectable channel.

The methods of the invention may be carried out by administering the composition containing substrates (including aglycone or glucuronidated form) of the invention by any route whereby drugs are conventionally administered. Such routes include systemic and local routes. Examples are intravenous, intramuscular, subcutaneous, intracranial, intraventricular, inhalation (Gradinaru et al., 1999 supra), intraperitoneal, as well as oral routes. Preferably, the method of the invention is carried out via oral or intravenous routes of administration.

In accordance with this invention, the substrates described herein (including aglycone or glucuronidated form) are useful in pharmaceutically acceptable oral modes. A preferred oral dosage form comprises tablets, capsules of hard or soft gelatin, methylcellulose or of another suitable material easily dissolved in the digestive tract. The oral dosages contemplated in accordance with the present invention will vary in accordance with the needs of the individual patient as determined by the prescribing physician. The preferred oral dosage form is capsules or tablets containing from 50 to 500 mg of a substrate of the invention.

Typical preparations for intravenous administration would be sterile aqueous solutions including water/buffered solutions. Intraveneous vehicles include fluid, nutrient and electrolyte replenishers. Preservatives and other additives may also be present such as antibiotics and antioxidants. Compositions for bolus i.v. administration may contain up to 10 mg/ml (10,000 mg/liter) of substrate described herein. Compositions for i.v. administration preferably contain from about 50 mg/liter to about 500 mg/liter of at least one substrate described herein.

In carrying out the method of the invention, substrate of the invention (including aglycone or glucuronidated form) is generally given to adults daily, preferably orally or intravenously, in an amount of from about 5 mg/kg to about 30 mg/kg daily, in single or divided doses, preferably from about 13 mg/kg to about 17 mg/kg daily, with the precise dosage being varied depending upon the needs of the patient. The doses will be adapted according to the patient and the pathology to be treated and are for example 1 mg-100 mg/day. In general, this therapy is carried out for a period of about three months. Alternatively, the method of the invention may be carried out prophylactically for an indefinite time in those patients who are have a high risk of suffering an acute neurotoxic event, such as a stroke. For the treatment of an acute neurotoxic event, the patient should be treated in accordance with the method of the invention as soon as possible after the diagnosis of the acute neurotoxic event, preferably within twelve hours, and most preferably within six hours, of the onset of the neurotoxic event. When the drug is administered orally, it is generally administered at regular intervals.

These drugs are notably administered orally or via an injectable channel.

According to one special embodiment, the UGT substrates (including aglycone or glucuronidated form) described in the present invention are modified with specific groups facilitating the passage of said UGT substrate throughout the blood-brain barrier (BBB). One strategy is lipidization, which involves the addition of lipid-like groups through modification of the hydrophilic part of the substrate structure. The resulting lipid-soluble substrates are transported through the BBB by accessing pores that transiently form within the lipid bilayer. Another strategy is the addition of hydrophobic groups to enhance BBB transfer by passive diffusion. For examples, addition of methyl groups or acetylation of amine groups increase lipophilicity and brain penetration. A further approach is to modify the substrate so that the compound has a structure that mimics a nutrient, thus giving it access to one of the specialized carrier-mediated transport systems within the BBB such as amino acid, hexose, vitamin and neuropeptide carriers. An alternative approach is the coupling of the substrates, via chemical linkers, to small synthetic peptide-vectors that cross the cellular membranes efficiently such as Pegelin or Penetratin (for general revue, see Temsamani et al., 2000, Pharm. Sci. Technol. Today, 3, 155-162).

According to another embodiment, the substrate or preparation of the invention (including aglycone or glucuronidated form) is administered to the patient in need in combination with a second compound or formulation aimed at facilitating the transfer throughout the BBB. For examples, the substrate or preparation of the invention is combined witha hypertonic solution, a biologically active agents such as bradykinin and angiotensin peptides, a vasoactive substance such as histamine, leukotrienes which have the ability of disrupting BBB transiently. According to the present invention, "combination" or "combined with" means that the substrate or preparation of the invention and the second compound or formulation aimed at facilitating the transfer throughout the BBB can be used simultaneously or consecutively or so as to be staggered over time. Simultaneously refers to a coadministration. In this case, these two essential components can be mixed to form a composition prior to being administered, or can be administered at the same time to the patient in need. It is also possible to administer them consecutively, that is to say one after the other, irrespective of which component is administered first. Finally, it is possible to use a mode of administration which is staggered over time or is intermittent and which stops and restarts at intervals which may or may not be regular. It is pointed out that the routes and sites of administration of the two components can be different. The time interval between the administration and the routes and sites of administration can be defined by the skilled person. It is possible to recommend an interval of from 10 min to 72 h, advantageously of from 30 min to 48 h, preferably of from 1 to 24 h and, very preferably, of from 1 to 6 h.

Pharmacogenomic analysis (see for example Ciotti et al., 1997, Pahrmacogenetics, 7, 485-495) have shown that polymorphisms in the UGT genes can result in affected enzyme activity and in lowering or increasing the patient reactivity towards UGT substrates. For example, missense mutations in the UGT1A6 have been identified (A541→G541 and A552→C552) which cause variation in the UGT1A6 activity and therefore variation in the rate of their substrate metabolization (for more detail, see Ciotti et al., 1997, Pharmacogenetics, 7, 485-495). Thus, according to another special embodiment, the treatment and/or prevention methods of the present invention further comprise a preliminary step consisting in establishing if the patient to be treated is presenting a genetic polymorphism resulting in less or improved UGT activity. More specifically, said preliminary step consists in determining by nucleotide sequence analysis of the patient genomic DNA if the UGT1A6 gene of said patient to be treated comprises at least one of the mutation A541→G541 (T181A mutation) or A552→C552 (R184S mutation). Details of said method are disclosed for example in Ciotti et al., 1997, Pharmacogenetics, 7, 485-495 the full content of which is incorporated hereby by reference.

All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the light of the teachings of this invention that certain changes and modifications may be made thereto without departing from the spirit or scope of the appended claims.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practised otherwise than as specifically described. Accordingly, those skilled in the art will recognize, or able to ascertain using no more than routine experimentation, many equivalents to the specific embodiments of the invention described specifically herein. Such equivalents are intended to be encompassed in the scope of the following claims.

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov/, http://www.infobiogen.fr/, http://www.fmi.ch/biology/resea rch_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness are given in Berks, TIBTECH 12 (1994), 352-364.

### EXAMPLES

*Primary cortical neuron cultures.* Cortices from foetal Wistar rats (E16) were dissected and maintained in PBS without calcium and magnesium. They were then treated in PBS containing 0.25% Trypsin for 15 min at 37°C and placed in a complete medium containing Horse Serum for inhibiting trypsin activity. The cortices were then dissociated in complete medium (Neurobasal, 2% Horse Serum, 2mM Glutamine, B27 supplement (1X), 100µg/ml Gentamicyn, 10µM β Mercaptoethanol). Cells were seeded at 10⁵ cells per well in a 96 well plate previously coated with Poly-Ornithine at 9µg/ml. The cultures were maintained at 37°C in a humidified incubator with 5% CO₂. At day 2, 5µM Cytosine Arabinofuranoside was added to the cell cultures in order to inhibit glial cell proliferation. At day 7, the primary cultures of neurons were available for further experiments.

*Assessment of neuron cell death*. Neuron cell death was estimated by measuring the release of the cytosolic enzyme, lactate dehydrogenase (LDH), into the medium of cell cultures. The LDH release quantification was performed using the colorimetric CytoTox96® nonradioactive assay (Promega). Briefly, 100µL of cell culture medium were removed and centrifuged for 4 min at 1500 rpm in order to remove cellular debris. 50µL were then added to 50µL of assay buffer and placed in the dark for 30min at room temperature. The reaction was stopped with 50µL of stop solution and the absorbance at 490nm was measured. The percentage of cell death was calculated by comparison with the control.

*Assessment of glutamate release.* Glutamate released by neurons in culture was evaluated in aliquots of the supernatants by means of a chemiluminescent enzymatic assay (Israel et al., Neurochem Int. 1993 Jan;22(1):53-8).

### EXAMPLE 1

### Neuroprotection assay.

Diazepam (Sigma) and the glucuronide derivative of Diazepam, named Temazepam (Alltech), a substrate of the UDP-glucuronosyltransferase 2B7 (UGT2B7), were used to illustrate the preventing and/or treating effects of UGT2B7 substrates on glutamate-induced neurological disorders.

Diazepam and Temazepam were used to protect primary cultures of neurons, isolated from the rat brain cortex, from neurotoxin-induced cell death.

The neurons were treated *in vitro* with 3-nitropropionic acid (3-NP, Sigma) at 400µM, a toxin which induces epileptic seizures in animal models, in absence or presence of Diazepam and Temazepam at 0.1, 1 and 5µM. After 24 hours, the cultures of cells were analysed for the level of toxin-evoked cell death by means of lactate dehydrogenase release quantification.

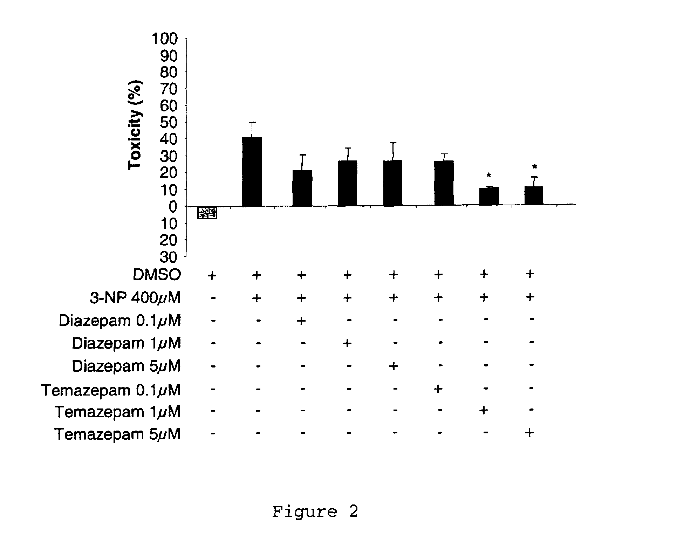

The results (Figure 2) show that Diazepam protects neurons from 3-NP at all tested doses but that Temazepam significantly reinforces said cell protection by about 50% at 1 and 5µM. These data indicate that glucuronide modification of Diazepam influences cell survival upon exposure to a toxic compound.

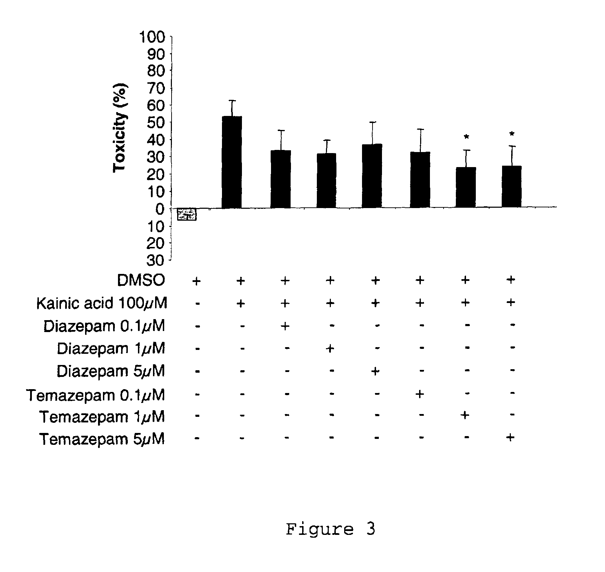

Similar results were obtained with another neurotoxin, the kainic acid at 100µM (Fisher Bioblock), which mimics glutamate-induced epileptic seizures in animal models. A better neuroprotection was observed with 1 and 5µM of Temazepam compared with Diazepam at the same concentrations (Figure 3).

### EXAMPLE 2

### Neuroprotection assay.

1-Naphthol (Merck), a substrate of UDP-glucuronosyltransferase 1A6 (UGT1A6), was used to illustrate the preventing and/or treating effects of UGT1A6 substrates on glutamate-induced neurological disorders.

1-Naphthol was used to protect primary cultures of neurons, isolated from the rat brain cortex, from neurotoxin-induced cell death.

The neurons were treated *in vitro* with 5µM of Ionomycin (Sigma), a calcium ionophore, in presence of 10µM of 1-Naphthol, in absence or in presence of 5 or 10µM of 3-methylcholanthrene (3-MC, Aldrich), an UGT inducer which allows the *in situ* glucuronidation of 1-Naphthol.

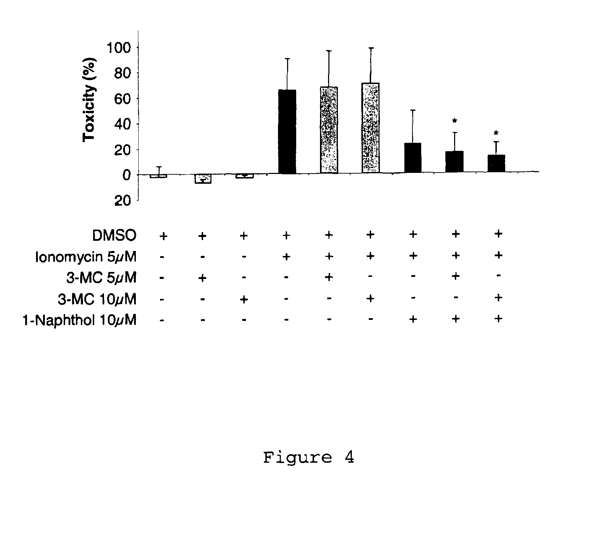

The results (Figure 4) show that Naphthol protects neurons from Ionomycin-evoked cell death and that glucuronidation of 1-Naphthol by 3-MC reinforces, dose dependently, the protective effect of 1-Naphthol.

These data indicate that glucuronide derivatives of UGT1A6 substrates present better neuroprotective effects than the parent molecules.

### Glutamate level determination.

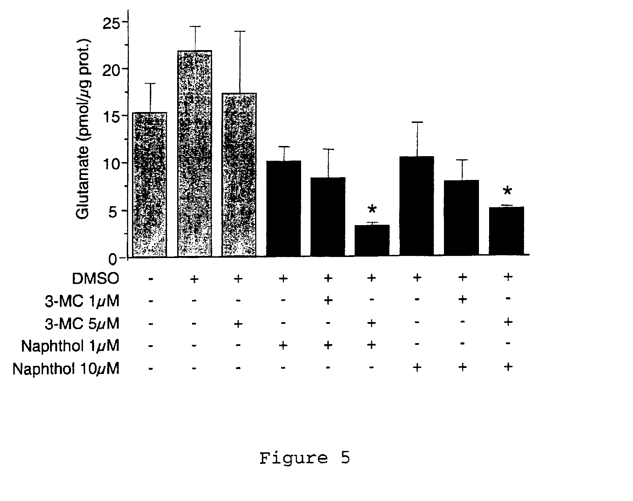

The glutamate released by neurons was followed by an enzymatic assay coupled to a chemiluminescent reaction. In this experiment, primary cultures of neurons isolated from the rat brain cortex, were treated by 1-Naphthol at 10µM with or without 3-MC at 1 or 5µM. The results (Figure 5) indicate that 1-Naphthol reduces glutamate release and that glucuronidation of 1-Naphthol by 3-MC reinforces, dose dependently, the decrease of glutamate.

This experiment indicates that the protective effect of glucuronidation on UGT1A6 substrates is associated with an improvement of the decrease of glutamate release.
Figure 1 (A/B) : Illustrates the chemical structures of designed compounds cited throughout the specification.
Figure 2 : Effect of Diazepam and Temazepam on neuron cell death. Neuron cell death in culture was arbitrarily set at 0. The cells were treated with the vehicle (lane 1) and with 3-NP at 400µM alone (lane 2) or simultaneously with one of the three tested concentrations of Diazepam (lanes 3-5) and Temazepam (lanes 6-8). Results indicate that Temazepam at 1µM (lane 7) and 5µM (lane 8) but not at 0.1µM (lane 6) improves the neuroprotective effect of Diazepam observed at all concentrations (lanes 3-5).
Figure 3 : Effect of Diazepam and Temazepam on neuron cell death. Neuron cell death in culture was arbitrarily set at 0. The cells were treated with the vehicle (lane 1) and with kainic acid at 100µM alone (lane 2) or simultaneously with one of the three tested concentrations of Diazepam (lanes 3-5) and Temazepam (lanes 6-8). Results indicate that Temazepam at 1µM (lane 7) and 5µM (lane 8) but not at 0.1µM (lane 6) improves the neuroprotective effect of Diazepam observed at all concentrations (lanes 3-5).
Figure 4 : Effect of 1-Naphthol and 1-Naphthol glucuronide on neuron cell death. Neuron cell death in culture was arbitrarily set at 0. The cells were treated with the vehicle (lane 1) and with Ionomycin at 5µM alone (lane 4) or simultaneously with 1-Naphtol at 10µM (lane 7) with 3-Methylcholanthrene at 5µM (lane 8) or 10µM (lane 9). The two tested doses of 3-Methylcholanthrene were evaluated in absence (lanes 2, 3) or presence (lanes 5, 6) of Ionomycin and considered as controls. Results indicate that 3-Methylcholanthrene alone has no effect on the natural death of neurons (lanes 2, 3) and that it has no protective effect against the toxicity induced by Ionomycin (lanes 5, 6). Results also show that the protection induced by 1-Naphthol at 10µM (lane 7) is improved by the glucuronidation induced by 3-Methylcholanthrene at 5µM (lane 8) and 10µM (lane 9).
Figure 5 : Effect of 1-Naphthol and 1-Naphthol glucuronide on glutamate released by neurons in culture. Glutamate release was evaluated by means of a chemiluminescent enzymatic assay. The basal levels of glutamate release were quantified in the non-treated cells (lane 1), in cells treated with the vehicle (lane 2) and in cells treated with 3-Methylcholanthrene at the highest dose tested in this experiment, 5µM (lane 3). The effect of 1-Naphthol at 1µM (lane 4) and 10µM (lane 7) was evaluated in presence of 3-Methylcholanthrene at 1µM (lanes 5, 8) and at 5µM (lanes 6, 9). Results indicate that the vehicle and the glucuronidation inducer 3-Methylcholanthrene have no effect on glutamate release (lanes 2, 3) whereas 1-Naphthol at both concentrations reduces the release of glutamate (lanes 4, 7). In addition, the glucuronidation of 1-Naphthol at both doses improves the decrease of glutamate for the two tested doses of 3-Methylcholanthrene (lanes 5, 6, 8, 9). This improvement becomes statistically significant at the highest dose of 3-Methylcholanthrene (lanes 6, 9) whatever the 1-Naphthol dose is.

## Claims

1. Use of at least one substrate of an UDP-glucuronosyltransferase (UGT), and salts thereof, for the preparation of a pharmaceutical composition having an inhibitory effect on the extracellular glutamate release into an individual treated with said composition.

2. The use of claim 1, wherein said UDP-glucuronosyltransferase (UGT) is expressed in brain and is selected among the group consisting of UDP-glucuronosyltransferase 1A6 (UGT1A6) and UDP-glucuronosyltransferase 2B7 (UGT2B7).

3. The use of claim 1 or 2, wherein said UGT substrate is selected among the group consisting of planar and small phenols, polycyclic aromatic hydrocarbons, and compounds which are structurally related.

4. The use of claims 1-3, wherein said substrate is a substrate of the UDP-glucuronosyltransferase 1A6 (UGT1A6) and is selected in the group consisting of 1-Naphthol, 2-Naphthol , 4-nitrophenol , methylsalicylate, ketoprofen , naproxen, 5-OH tryptamine/serotonin, carprofen/rimadyl , acetaminophen/paracetamol ,benzidine, 4-methylumbelliferone (4-MU), silymarin.

5. The use of claims 1-3, wherein said substrate is a substrate of the UDP-glucuronosyltransferase 2B7 (UGT2B7) and is selected in the group consisting of transretinoic acid, ASA , AZT , benoxaprofen , benzidine, (benzo(a)pyrene mbs), buprenorphine, carprofen, chloramphenicol, clofibric acid , flavenoids quercetin, kaempfenol, cyclosporin, DMXAA, diclofenac, dihydrocodeine DHC, dihydromorphone, mefenamic acid, fenemate NSAID, mycophenolic acid MCPA mofetil, fenoprofen, hydromorphone, ibuprofen, ketoprofen, linoleic acid, lorazepam, losartan, menthol, morphine 3 and morphine 6, nalbufene, nalmefene, naltrindole, nalorphine, naloxone, naltrexone, S-naproxen, norcodeine, normorphone, oxycodone, oxymorphone, pirprofen, propanolol, S-oxazepam, tacrolimus, temazepam, tolcapone, tiaprofenic, valproate, zomepirac, 5-OH tryptamine.

6. The use of claims 1-5, wherein said pharmaceutical composition is intended for treating and/or preventing glutamate-evoked cytotoxicity in a patient in need.

7. The use of claims 1-5, wherein said pharmaceutical composition is intended for treating and/or preventing glutamate-evoked neurotoxicity.

8. The use of claims 1-5, wherein said pharmaceutical composition is intended for treating and/or preventing neurodegeneration.

9. The use of claims 1-5, wherein said pharmaceutical composition is intended for modulating the release of glutamate in a patient.

10. The use of claims 1-5, wherein said pharmaceutical composition is intended for treating and/or preventing disease and/or condition associated with the excessive release of glutamate in a patient.

11. The use of claim 10, wherein said disease and/or condition associated with the excessive release of glutamate is selected among the group consisting of epileptic seizures, acute and chronic neurodegenerative diseases, ischemia, Alzheimer's, Huntington's, Parkinson's diseases, multiple sclerosis (MS), amyotrophic lateral sclerosis (ALS), spinal muscular atrophy (SMA), retinopathy, stroke and traumatic brain injury, drug-induced neurotoxicity, pain, hormonal balance, blood pressure, thermoregulation, respiration, learning, pattern recognition, memory, and disorders subsequent to hypoxia or hypoglycaemia.

12. Use of at least one glucuronidated substrate of an UDP-glucuronosyltransferase (UGT) and salts thereof, for the preparation of a pharmaceutical composition having an inhibitory effect on the extracellular glutamate release into an individual treated with said composition.

13. The use of claim 12, wherein said glucuronidated UGT substrate is selected among the group consisting of planar and small glucuronidated phenols, polycyclic aromatic glucuronidated hydrocarbons, and compounds which are structurally related.

14. The use of claims 12-13, wherein said glucuronidated substrate is selected in the group consisting of 1-Naphthol, 2-Naphthol , 4-nitrophenol , methylsalicylate, ketoprofen , naproxen, 5-OH tryptamine/serotonin, carprofen/rimadyl , acetaminophen/paracetamol ,benzidine, 4-methylumbelliferone (4-MU), silymarin and is further glucuronidated.

15. The use of claims 12-13, wherein said glucuronidated substrate is selected in the group consisting of transretinoic acid, ASA , AZT , benoxaprofen , benzidine, (benzo(a)pyrene mbs), buprenorphine, carprofen, chloramphenicol, clofibric acid , flavenoids quercetin, kaempfenol, cyclosporin, DMXAA, diclofenac, dihydrocodeine DHC, dihydromorphone, mefenamic acid, fenemate NSAID, mycophenolic acid MCPA mofetil, fenoprofen, hydromorphone, ibuprofen, ketoprofen, linoleic acid , lorazepam, losartan, menthol, morphine 3 and morphine 6, nalbufene, nalmefene, naltrindole, nalorphine, naloxone, naltrexone, S-naproxen, norcodeine, normorphone, oxycodone, oxymorphone, pirprofen, propanolol, S-oxazepam, tacrolimus, temazepam, tolcapone, tiaprofenic, valproate, zomepirac, 5-OH tryptamine and is further glucuronidated.


SEQUENCE LISTING

(1) GENERAL INFORMATION:
(iii) NUMBER OF SEQUENCES: 2
(2) INFORMATION FOR SEQ ID NO: 1:
(i) SEQUENCE CHARACTERISTICS:
(A) LENGTH: 531 amino acids
(B) TYPE: amino acid
(C) STRANDEDNESS:
(D) TOPOLOGY: linear
(ii) MOLECULE TYPE: protein
(iii) HYPOTHETICAL: NO
(iv) ANTI-SENSE: NO
(vi) ORIGINAL SOURCE:
(A) ORGANISM: UDP glycosyltransferase 1 family,
polypeptide A6 [Homo sapiens]

(ix) FEATURE:
(A) NAME/KEY: UDP glycosyltransferase 1A6
(B) LOCATION:

(xi) SEQUENCE DESCRIPTION: SEQ ID NO:1

1 MACLLRSFQR ISAGVFFLAL WGMVVGDKLL VVPQDGSHWL SMKDIVEVLS
DRGHEIVVVV
61 PEVNLLLKEY KYYTRKIYPV PYDQEELKNR YQSFGNNHFA ERSFLTAPQT
EYRNNMIVIG
121 LYFINCQSLL QDRDTLNFFK ESKFDALFTD PALPCGVILA EYLGLPSVYL
FRGFPCSLEH
181 TFSRSPDPVS YIPRCYTKFS DHMTFSQRVA NFLVNLLEPY LFYCLFSKYE
KLASAVLKRD
241 VDIITLSEVS VWLLRYDFVL EYPRPVMPNM VFIGGINCKK RKDLSQEFEA
YINASGEHGI
301 VVFSLGSMVS EIPEKKAMAI ADALGKNPQT VLWRYTGTRP SNLANNTILV
KWLPQNDLLG
361 HPMTRAFITH AGSHGVYESI CNGVPMVMMP LFGDQMDNAK RMETKGAGVT
LNVLEMTSED
421 LENALKAVIN DKSYKENIMR LSSLHKDRPV EPLDLAVFWV EFVMRHKGAP
HLRPAAHDLT

481 WYQYHSLDVI GFLLAVVLTV AFITFKCCPY GYPKCLGKKG RVKKAHKSKT
H

(3) INFORMATION FOR SEQ ID NO: 2:
(i) SEQUENCE CHARACTERISTICS:
(A) LENGTH: 529 amino acids
(B) TYPE: amino acid
(C) STRANDEDNESS:
(D) TOPOLOGY: linear
(ii) MOLECULE TYPE: protein
(iii) HYPOTHETICAL: NO
(iv) ANTI-SENSE: NO
(vi) ORIGINAL SOURCE:
(A) ORGANISM: UDP-glucuronyltransferase, family
2, beta-7 [Homo sapiens]

(ix) FEATURE:
(A) NAME/KEY: UDP glycosyltransferase 2B7
(B) LOCATION:

(xi) SEQUENCE DESCRIPTION: SEQ ID NO:2

1 MSVKWTSVIL LIQLSFCFSS GNCGKVLVWA AEYSHWMNIK TILDELIQRG
HEVTVLASSA
61 SILFDPNNSS ALKIEIYPTS LTKTELENFI MQQIKRWSDL PKDTFWLYFS
QVQEIMSIFG
121 DITRKFCKDV VSNKKFMKKV QESRFDVIFA DAIFPCSELL AELFNIPFVY
SLSFSPGYTF
181 EKHSGGFIFP PSYVPVVMSE LTDQMTFMER VKNMIYVLYF DFWFEIFDMK
KWDQFYSEVL
241 GRPTTLSETM GKADVWLIRN SWNFQFPHPL LPNVDFVGGL HCKPAKPLPK
EMEDFVQSSG
301 ENGVVVFSLG SMVSNMTEER ANVIASALAQ IPQKVLWRFD GNKPDTLGLN
TRLYKWIPQN
361 DLLGHPKTRA FITHGGANGI YEAIYHGIPM VGIPLFADQP DNIAHMKARG
AAVRVDFNTM
421 SSTDLLNALK RVINDPSYKE NVMKLSRIQH DQPVKPLDRA VFWIEFVMRH
KGAKHLRVAA

481 HDLTWFQYHS LDVIGFLLVC VATVIFIVTK CCLFCFWKFA RKAKKGKND

SEQUENCE LISTING

<110>  FAUST PHARMACEUTICALS

<120>  Methods for the prevention and/or the treatment of neurological
disorders

<130>  H1870 EP S3

<140>  EP 03 01 1017.5

<141>  2003-05-16

<150>  EP 02291218.2

<151>  2002-05-17

<150>  EP 02291600.1

<151>  2002-06-27

<150>  US 60/393,108

<151>  2002-07-03

<160>  2

<170>  PatentIn version 3.1

<210>  1

<211>  531

<212>  PRT

<213>  Homo sapiens

<220>

<221>  MISC_FEATURE

<223>  UDP glycosyltransferase 1 family, polypeptide A6

<400>  1

Met Ala Cys Leu Leu Arg Ser Phe Gln Arg Ile Ser Ala Gly Val Phe
1               5                   10                  15

Phe Leu Ala Leu Trp Gly Met Val Val Gly Asp Lys Leu Leu Val Val
            20                  25                  30

Pro Gln Asp Gly Ser His Trp Leu Ser Met Lys Asp Ile Val Glu Val
        35                  40                  45

Gly Thr Arg Pro Ser Asn Leu Ala Asn Asn Thr Ile Leu Val Lys Trp
            340                 345                 350

Leu Pro Gln Asn Asp Leu Leu Gly His Pro Met Thr Arg Ala Phe Ile
        355                 360                 365

Thr His Ala Gly Ser His Gly Val Tyr Glu Ser Ile Cys Asn Gly Val
    370                 375                 380

Pro Met Val Met Met Pro Leu Phe Gly Asp Gln Met Asp Asn Ala Lys
385                 390                 395                 400

Arg Met Glu Thr Lys Gly Ala Gly Val Thr Leu Asn Val Leu Glu Met
                405                 410                 415

Thr Ser Glu Asp Leu Glu Asn Ala Leu Lys Ala Val Ile Asn Asp Lys
            420                 425                 430

Ser Tyr Lys Glu Asn Ile Met Arg Leu Ser Ser Leu His Lys Asp Arg
        435                 440                 445

Pro Val Glu Pro Leu Asp Leu Ala Val Phe Trp Val Glu Phe Val Met
    450                 455                 460

Arg His Lys Gly Ala Pro His Leu Arg Pro Ala Ala His Asp Leu Thr
465                 470                 475                 480

Trp Tyr Gln Tyr His Ser Leu Asp Val Ile Gly Phe Leu Leu Ala Val
                485                 490                 495

Val Leu Thr Val Ala Phe Ile Thr Phe Lys Cys Cys Pro Tyr Gly Tyr
            500                 505                 510

Pro Lys Cys Leu Gly Lys Lys Gly Arg Val Lys Lys Ala His Lys Ser
        515                 520                 525

Lys Thr His
    530

<210>  2

<211>  529

<212>  PRT

<213>  Homo sapiens


<220>

<221>  MISC_FEATURE

<223>  UDP-glucuronyltransferase, family 2, beta-7

Leu Ser Asp Arg Gly His Glu Ile Val Val Val Val Pro Glu Val Asn
    50                  55                  60

Leu Leu Leu Lys Glu Tyr Lys Tyr Tyr Thr Arg Lys Ile Tyr Pro Val
65                  70                  75                  80

Pro Tyr Asp Gln Glu Glu Leu Lys Asn Arg Tyr Gln Ser Phe Gly Asn
                85                  90                  95

Asn His Phe Ala Glu Arg Ser Phe Leu Thr Ala Pro Gln Thr Glu Tyr
            100                 105                 110

Arg Asn Asn Met Ile Val Ile Gly Leu Tyr Phe Ile Asn Cys Gln Ser
        115                 120                 125

Leu Leu Gln Asp Arg Asp Thr Leu Asn Phe Phe Lys Glu Ser Lys Phe
    130                 135                 140

Asp Ala Leu Phe Thr Asp Pro Ala Leu Pro Cys Gly Val Ile Leu Ala
145                 150                 155                 160

Glu Tyr Leu Gly Leu Pro Ser Val Tyr Leu Phe Arg Gly Phe Pro Cys
                165                 170                 175

Ser Leu Glu His Thr Phe Ser Arg Ser Pro Asp Pro Val Ser Tyr Ile
            180                 185                 190

Pro Arg Cys Tyr Thr Lys Phe Ser Asp His Met Thr Phe Ser Gln Arg
        195                 200                 205

Val Ala Asn Phe Leu Val Asn Leu Leu Glu Pro Tyr Leu Phe Tyr Cys
    210                 215                 220

Leu Phe Ser Lys Tyr Glu Lys Leu Ala Ser Ala Val Leu Lys Arg Asp
225                 230                 235                 240

Val Asp Ile Ile Thr Leu Ser Glu Val Ser Val Trp Leu Leu Arg Tyr
                245                 250                 255

Asp Phe Val Leu Glu Tyr Pro Arg Pro Val Met Pro Asn Met Val Phe
            260                 265                 270

Ile Gly Gly Ile Asn Cys Lys Lys Arg Lys Asp Leu Ser Gln Glu Phe
        275                 280                 285

Glu Ala Tyr Ile Asn Ala Ser Gly Glu His Gly Ile Val Val Phe Ser
    290                 295                 300

Leu Gly Ser Met Val Ser Glu Ile Pro Glu Lys Lys Ala Met Ala Ile
305                 310                 315                 320

Ala Asp Ala Leu Gly Lys Asn Pro Gln Thr Val Leu Trp Arg Tyr Thr
                325                 330                 335

Asn Val Asp Phe Val Gly Gly Leu His Cys Lys Pro Ala Lys Pro Leu
        275                 280                 285

Pro Lys Glu Met Glu Asp Phe Val Gln Ser Ser Gly Glu Asn Gly Val
    290                 295                 300

Val Val Phe Ser Leu Gly Ser Met Val Ser Asn Met Thr Glu Glu Arg
305                 310                 315                 320

Ala Asn Val Ile Ala Ser Ala Leu Ala Gln Ile Pro Gln Lys Val Leu
                325                 330                 335

Trp Arg Phe Asp Gly Asn Lys Pro Asp Thr Leu Gly Leu Asn Thr Arg
            340                 345                 350

Leu Tyr Lys Trp Ile Pro Gln Asn Asp Leu Leu Gly His Pro Lys Thr
        355                 360                 365

Arg Ala Phe Ile Thr His Gly Gly Ala Asn Gly Ile Tyr Glu Ala Ile
    370                 375                 380

Tyr His Gly Ile Pro Met Val Gly Ile Pro Leu Phe Ala Asp Gln Pro
385                 390                 395                 400

Asp Asn Ile Ala His Met Lys Ala Arg Gly Ala Ala Val Arg Val Asp
                405                 410                 415

Phe Asn Thr Met Ser Ser Thr Asp Leu Leu Asn Ala Leu Lys Arg Val
            420                 425                 430

Ile Asn Asp Pro Ser Tyr Lys Glu Asn Val Met Lys Leu Ser Arg Ile
        435                 440                 445

Gln His Asp Gln Pro Val Lys Pro Leu Asp Arg Ala Val Phe Trp Ile
    450                 455                 460

Glu Phe Val Met Arg His Lys Gly Ala Lys His Leu Arg Val Ala Ala
465                 470                 475                 480

His Asp Leu Thr Trp Phe Gln Tyr His Ser Leu Asp Val Ile Gly Phe
                485                 490                 495

Leu Leu Val Cys Val Ala Thr Val Ile Phe Ile Val Thr Lys Cys Cys
            500                 505                 510

Leu Phe Cys Phe Trp Lys Phe Ala Arg Lys Ala Lys Lys Gly Lys Asn
        515                 520                 525

Asp

<400> 2

Met Ser Val Lys Trp Thr Ser Val Ile Leu Leu Ile Gln Leu Ser Phe
1               5                   10                  15

Cys Phe Ser Ser Gly Asn Cys Gly Lys Val Leu Val Trp Ala Ala Glu
            20                  25                  30

Tyr Ser His Trp Met Asn Ile Lys Thr Ile Leu Asp Glu Leu Ile Gln
        35                  40                  45

Arg Gly His Glu Val Thr Val Leu Ala Ser Ser Ala Ser Ile Leu Phe
    50                  55                  60

Asp Pro Asn Asn Ser Ser Ala Leu Lys Ile Glu Ile Tyr Pro Thr Ser
65                  70                  75                  80

Leu Thr Lys Thr Glu Leu Glu Asn Phe Ile Met Gln Gln Ile Lys Arg
                85                  90                  95

Trp Ser Asp Leu Pro Lys Asp Thr Phe Trp Leu Tyr Phe Ser Gln Val
            100                 105                 110

Gln Glu Ile Met Ser Ile Phe Gly Asp Ile Thr Arg Lys Phe Cys Lys
        115                 120                 125

Asp Val Val Ser Asn Lys Lys Phe Met Lys Lys Val Gln Glu Ser Arg
    130                 135                 140

Phe Asp Val Ile Phe Ala Asp Ala Ile Phe Pro Cys Ser Glu Leu Leu
145                 150                 155                 160

Ala Glu Leu Phe Asn Ile Pro Phe Val Tyr Ser Leu Ser Phe Ser Pro
                165                 170                 175

Gly Tyr Thr Phe Glu Lys His Ser Gly Gly Phe Ile Phe Pro Pro Ser
            180                 185                 190

Tyr Val Pro Val Val Met Ser Glu Leu Thr Asp Gln Met Thr Phe Met
        195                 200                 205

Glu Arg Val Lys Asn Met Ile Tyr Val Leu Tyr Phe Asp Phe Trp Phe
    210                 215                 220

Glu Ile Phe Asp Met Lys Lys Trp Asp Gln Phe Tyr Ser Glu Val Leu
225                 230                 235                 240

Gly Arg Pro Thr Thr Leu Ser Glu Thr Met Gly Lys Ala Asp Val Trp
                245                 250                 255

Leu Ile Arg Asn Ser Trp Asn Phe Gln Phe Pro His Pro Leu Leu Pro
            260                 265                 270